(19) **Europäisches Patentamt**
**European Patent Office**
**Office européen des brevets**

(11) Numéro de publication : **0 283 408 B1**

## (12) FASCICULE DE BREVET EUROPEEN

(45) Date de publication du fascicule du brevet :
**08.01.92 Bulletin 92/02**

(51) Int. Cl.⁵ : **C08L 83/04, A61K 9/22,**
**C08K 13/02, A61K 33/18,**
**C02F 1/76**

(21) Numéro de dépôt : **88420064.3**

(22) Date de dépôt : **23.02.88**

(54) **Composition élastomère silicone polycondensation contenant de l'iode.**

Le dossier contient des informations techniques présentées postérieurement au dépôt de la demande et ne figurant pas dans le présent fascicule.

(30) Priorité : **26.02.87 FR 8702883**

(43) Date de publication de la demande :
**21.09.88 Bulletin 88/38**

(45) Mention de la délivrance du brevet :
**08.01.92 Bulletin 92/02**

(84) Etats contractants désignés :
**AT BE CH DE ES FR GB GR IT LI LU NL SE**

(56) Documents cités :
**DE-A- 1 467 861**
**GB-A- 1 412 970**
**US-A- 2 918 400**
**US-A- 4 275 194**
**US-A- 4 384 960**
**US-A- 4 420 590**
**US-A- 4 640 956**

(73) Titulaire : **RHONE-POULENC CHIMIE**
**25, quai Paul Doumer**
**F-92408 Courbevoie Cédex (FR)**

(72) Inventeur : **Prazuck, Thierry**
**53, avenue Mathurin Moreau**
**F-75019 Paris (FR)**
Inventeur : **Torres, Ghislaine**
**104, rue Ney**
**F-69006 Lyon (FR)**
Inventeur : **Cyprien, Guy**
**7, rue d'Anjou**
**F-94240 L'Hay Les Roses (FR)**
Inventeur : **Fisch, Alain**
**22, rue de l'Yvette**
**F-75016 Paris (FR)**
Inventeur : **Haggiage, Johnny**
**72, rue du Fort Saint-Irénée**
**F-69005 Lyon (FR)**
Inventeur : **Porte, Hugues**
**6, chemin de Crépieux**
**F-69300 Caluire (FR)**

(74) Mandataire : **Fabre, Madeleine-France et al**
**RHONE-POULENC CHIMIE Direction des**
**Brevets Secteur Spécialités Chimiques 25,**
**quai Paul-Doumer**
**F-92408 Courbevoie Cédex (FR)**

EP 0 283 408 B1

## Description

La présente invention concerne une composition élastomère de silicone réticulant par réaction de polycondensation contenant de l'iode ainsi qu'un procédé de traitement des eaux d'usage domestique et de boissons à l'aide de cette composition.

On estime actuellement à plusieurs centaines de millions le nombre de sujets présentant une déficience ou une carence en iode dans le monde. Les zones géographiques les plus touchées sont l'Amérique Latine, en particulier le long de la Cordillière des Andes, la quasi totalité des pays non côtiers de l'Afrique et de l'Asie (Pakistan, Inde, Népal, Chine, Laos, etc.....).

Les principales conséquences pathologiques de la déficience en iode sont bien connues. Il s'agit essentiellement d'une part du goître et ses complications parmi lesquelles on peut citer les troubles de la déglutition, les troubles respiratoires, la cancérisation, la circulation collatérale, et, d'autre part l'hypothyroïdie et ses complications parmi lesquelles on peut citer : le crétinisme, les désordres cérébraux, les accouchements prématurés, les fausses couches et les anomalies congénitales.

Si la carence en iode a disparu des pays industrialisés grâce à l'iodation du sel de cuisine, il n'en est pas de même dans les pays en voie de développement où les deux principales actions menées jusqu'àlors sont demeurées inefficaces.

Ces actions visent essentiellement d'une part :

— l'iodation du sel de cuisine : elle est inopérante dans la plupart des pays en voie de développement car bien souvent la consommation de sel est minime, les circuits de distribution du sel à travers des réseaux économiques et commerciaux sont quasiment inexistants et, enfin, en région tropicale, l'iode rajouté au sel s'en échappe rapidement lorsqu'il n'est pas parfaitement emballé.

et d'autre part :

— l'injection intra-musculaire de l'huile iodée : cette injection a l'avantage de présenter une action différée (action retard) mais elle n'est pas sans présenter des inconvénients, en particulier les risques d'infection, les risques d'allergie à l'iode, les risques d'hyperthyroïdie ou d'hypothyroïdie induite par l'injection d'une dose nécessairement supra-physiologique.

On a par ailleurs décrit dans le brevet belge BE-A-889680 l'introduction d'oligo-éléments, dont l'iode, dans l'eau de boisson des ruminants sous la forme d'une dispersion dans un liant comme par exemple du plâtre de Paris. Un diorganopolysiloxane peut être ajouté en vue de retarder la diffusion de l'oligo-élément. En outre l'utilisation d'iode et de composés de l'iode pour désinfecter ou pour purifier l'eau est bien connue. On peut citer à titre d'exemple les brevets américains US-A2347567, US-A-2743208 et US-A-3408295.

Il existe également de très nombreux brevets décrivant l'utilisation de systèmes polymères en particulier de silicone pour la libération contrôlée d'un principe actif au moyen par exemple d'un système transdermique, (brevet américain US-A-4053580) ou par absorption buccale notamment pour des ruminants (brevet français FR-A-2560768).

Enfin par le brevet américain US-A-4384960 il est décrit la mise en pastilles d'iode $I_2$ dans une bouteille en plastique dans laquelle l'eau pénètre par une membrane. L'eau solubilise l'iode. Le rôle de la membrane est seulement d'éviter que l'iode en pastilles ne sorte pas de la bouteille.

Il est simplement suggéré en outre qu'il est possible d'introduire l'iode $I_2$ dans la bouteille au sein d'une dispersion liquide de silicone ou d'un élastomère de diméthylsiloxane puis de les faire durcir. Cette solution indiquée n'est pas techniquement réalisable car $I_2$ est un inhibiteur bien connu des catalyseurs de durcissement des élastomères silicones vulcanisables à température ambiante (voir en particulier la publication de W.D. MORAIN et al. Plastic and Reconstructive Surgery 59, 2, 215-222 (1977).

En outre dans le système du brevet US-A-4 384 960 il n'y a pas de contrôle de la libération de l'iode d'une part, et, d'autre part, l'iodation de l'eau se fait par addition discontinue ou en continu de quelques gouttes d'eau très iodée (à saturation) contenue dans la bouteille, dans un récipient quelconque contenant de l'eau non traitée. Il est clair que la solution proposée par US-A-4384960 est imparfaite par le fait notamment qu'il s'agit d'une technique individuelle qui, comme l'injection intra-musculaire d'iode, nécessite une éducation et une mobilisation massive des populations.

Le brevet US-A-4420590 enseigne que de l'eau peut être rendue potable après passage au contact d'une résine échangeuse d'anion traitée par essentiellement :

— de l'iode élémentaire,

— du bromure de potassium,

— de l'iodure de potassium,

dans des proportions bien définies

Le brevet US-A-4384960 enseigne qu'il est possible de prévenir les conséquences d'une carence en iode en ajoutant de l'iode élémentaire (c'est-à-dire du degré d'oxydation zéro) à de l'eau.

2

La demande allemande DE-A-1467861 enseigne que des médicaments, tels que par exemple des hormones, vitamines, antibiotiques, anticoagulants, peuvent être introduits dans l'organisme alors qu'ils se trouvent dans des élastomères silicones.

La présente invention a précisément pour but de proposer une composition élastomère de silicone polycondensation contenant de l'iode utilisable pour le traitement continu des eaux d'usage domestique, en particulier dans les systèmes d'adduction, de traitement des eaux dans les puits et les forages et qui permette de relarguer (libérer) une quantité contrôlée et adaptée d'iode en vue d'assurer l'éradication des diverses maladies dues à une carence en iode.

Elle a également pour but de proposer une composition élastomère de silicone polycondensation contenant de l'iode qui immergée de façon appropriée dans les endroits contenant l'eau à traiter, en particulier les puits et forages, relargue (libère) de façon continue, de préférence pendant au moins un an, une quantité appropriée d'iode sous une forme et à une dose thérapeutiquement active et efficace pour soigner les diverses maladies dues à une carence en iode.

Elle a également pour but de proposer une composition élastomère de silicone polycondensation contenant de l'iode qui, immergée de façon appropriée dans les endroits contenant l'eau à traiter n'ait aucune action secondaire indésirable et néfaste sur le plan chimique et biologique sur les eaux à traiter.

Elle a également pour but de proposer une composition élastomère de silicone polycondensation contenant de l'iode présentant une forme adaptée au milieu dans lequel se trouve l'eau à traiter, cette forme étant adaptée en particulier aux puits et/ou forages et présentant un système d'introduction dans les puits et/ou forages permettant son remplacement aisé.

Elle a également pour but de proposer une composition élastomère de silicone polycondensation contenant de l'iode apporté par un composé de l'iode qui n'inhibe pas le durcissement (réticulation) de la composition de silicone polycondensation en un élastomère.

Ces buts et d'autres sont atteints par la présente invention qui concerne en effet une composition diorganopolysiloxane durcissable en un élastomère de silicone par des réactions de polycondensation comportant :

(A) : au moins une huile diorganopolysiloxane portant à chaque extrémité de la chaîne au moins deux groupes condensables ou hydrolysables, ou un seul groupe hydroxy, ladite huile ayant une viscosité à 25°C inférieure à 1000000 mPa · s,

(B) : au moins un composé de l'iode, à l'exclusion de l'iode moléculaire $I_2$, solide à température ambiante, soluble dans l'eau, non toxique dispersé de façon homogène dans la composition et n'inhibant pas le durcissement de la composition en un élastomère,

(C) : un catalyseur de polycondensation de l'huile,

(D) : un silane comportant au moins trois groupes condensables ou hydrolysables, notamment quand (A) est une huile à extrémités hydroxy.

On peut introduire de 5 à 130 parties, de préférence de 10 à 100 parties de composé d'iode (B) pour 100 parties de l'huile (A).

Dans ce qui suit ou ce qui précède, sauf mentions contraires, les pourcentages et parties sont en poids.

Comme composé de l'iode on peut utiliser :

— les iodures ou iodates de formules générales :

$$(M^{a+}) (I)_a$$
$$\text{et} \quad (M^{a+}) (IO_3^-)_a$$

dans lesquelles a est un nombre entier supérieur ou égal à 1 et M est un cation qui peut être choisi parmi un métal alcalin tel que le sodium et le potassium, un métal alcalino-terreux tel que le magnésium et le calcium, un métal de transition tel que le fer et le manganèse, un ammonium quaternaire $(NZ_4)^+$, dont les radicaux Z identiques ou différents représentent un radical alkyle linéaire ou ramifié en $C_1$-$C_{20}$ ou un atome d'hydrogène, tel que l'ion ammonium $NH^+_4$.

Les cations $M^{a+}$ et $NZ_4^+$, sont choisis de telle sorte que l'iodure ou l'iodate correspondant soit un solide ou un liquide à température ambiante, soit soluble dans l'eau et soit non toxique.

Comme iodures et iodates on peut en particulier utiliser ceux de formules :

Na I, Na $IO_3$,

K I, K $IO_3$,

Mg $I_2$, Mg $I_2 \cdot 8H_2O$,

$Mg(IO_3)_2 \cdot 4H_2O$,

$NH_4$ I

Fe $I_2 \cdot 4H_2O$

Mn $I_2$

Ces sels peuvent contenir de l'eau d'hydratation ou de l'eau de constitution.

Pour des raisons de facilité de mise en oeuvre les composés d'iode solides sont préférés, et parmi ceux-ci $NaI$ et $KIO_3$ sont les plus préférés.

Tous les composés de l'iode tels que définis ci-dessus, lorsqu'ils sont en solution dans l'eau à traiter, libèrent l'iode sous une forme non toxique et thérapeutiquement efficace. Par composé de l'iode non toxique on entend selon l'invention un composé qui, en solution, n'est pas toxique aux posologies préconisées par la présente invention.

Par composé de l'iode soluble dans l'eau on entend un composé présentant une solubilité d'au moins 100 µg/l à température ambiante.

Par ailleurs les composés de l'iode ne doivent pas inhiber le durcissement de la composition de silicone en élastomère. L'iode moléculaire $I_2$ est donc exclu des composés de l'iode utilisable dans le cadre de la présente invention.

En particulier dans les pays en développement, l'eau à usage domestique (boisson, lavage, irrigation, etc.....) est pour l'essentiel àapportée par deux types de structure, les puits et les forages.

Pour des raisons évidentes de coût, d'efficacité, de salubrité, la création nouvelle d'un point d'eau se fait souvent par forage.

Un forage est une colonne d'air forée à travers des roches compactes ayant une profondeur comprise généralement entre 20 et 100 mètres et un diamètre d'au moins environ 10 cm. L'eau s'infiltre dans cette colonne, par les fissures ou les divers interstices. La réserve d'eau immédiatement disponible est donc constituée d'une colonne de 10 à 70 mètres, généralement de 30 à 50 mètres de haut qui est extraite à l'aide d'une pompe à corps immergé.

Cette eau se renouvelle principalement en fonction de l'utilisation du forage qui de la saison. En effet en saison des pluies le forage est traditionnellement moins utilisé. Par contre en saison sèche, le forage débite environ 12 heures par jour, soit une quantité comprise entre 5 et 10 m³ par jour pendant environ six mois.

En règle générale, un puits peut être asséché deux fois durant la journée au moment de la saison sèche ce qui correspond avec ces données statistiques moyennes, à un maximum d'utilisation de 5 à 10 m³.

De nombreuses études montrent que dans les zones de grande endémie goîtreuse, le taux pré-existant d'équivalent en iode dans l'eau des forages ou des puits est inférieur à 2 microgrammes par litre (2 µg/l). Il est estimé actuellement qu'un apport journalier d'environ 100 µg d'équivalent iode par jour et par personne serait suffisant pour prévenir le développement d'un goître endémique et sans doute environ 150 µg lorsqu'il y a consommation régulière de substance goitrigènes. A l'inverse une intoxication aigüe à l'iode peut être responsable d'irritation neurologique, d'hyperthyroïdie ou d'hypothyroïdie.

Il est admis en pratique médicale que l'absorption d'une dose de 3 grammes d'équivalent d'iode par un sujet adulte en une seule prise n'entraîne pas d'effet secondaire.

Par conséquent, pouvoir apporter à un individu de 20 à 200 µg, de préférence environ 100 µg, d'équivalent iode par jour constitue le but recherché.

Ainsi sachant qu'un individu adulte absorbe en moyenne 2 litres d'eau par jour et sur la base des données ci-dessus (forage ayant un débit de 600 l/h), il apparaît souhaitable qu'un litre d'eau traitée contienne environ 50 µg/l d'iode, ce qui correspond à 50 µg d'équivalent d'iode par litre et par personne, ce qui nécessite que la composition de silicone relargue 720 mg/j d'équivalent d'iode, soit 270 g d'équivalent iode à relarguer pendant une année.

Dans ce qui suit ou ce qui précède, sauf indications contraires, les parties et pourcentages sont en poids.

De façon surprenante et inattendue on a en effet découvert selon la présente invention qu'il est possible d'ajouter dans une composition élastomère silicone polycondensation des quantités importantes de composé d'iode sous une forme solide ou liquide tel que défini ci-dessus, à savoir p. ex. de 5 à 130 parties, de préférence de 10 à 100 pour 100 parties de l'huile diorganopolysiloxane (A) éventuellement préalablement chargée avec une charge siliceuse renforçante ou semi-renforçante et d'obtenir ainsi un élastomère réticulé qui présente des caractéristiques mécaniques suffisantes pour l'application envisagée et qui, immergé dans l'eau permette d'assurer un relargage continu et contrôlé d'iode de préférence pendant au moins un an.

Le système de libération contrôlée de l'iode fait partie des systèmes matriciels dont la diffusion du principe actif est normalement régie par la loid de FICK, c'est-à-dire par une cinétique de diffusion d'ordre 1/2 pour seulement 60% en poids du principe actif. Au-delà de 60% il y a épuisement de la matrice et les flux de diffusion sont fortement diminués. De façon surprenante et inattendue, on a trouvé que le système matriciel silicone conforme à l'invention libère l'iode suivant une cinétique d'ordre 0 et de façon continue et cela jusqu'à ce que 80% en poids et plus du composé d'iode soit libéré.

L'avantage considérable apporté par la matrice silicone est donc qu'il est très facile d'extrapoler la diffusion continue du principe actif après une mesure de la quantité libérée au bout d'au moins un mois puisque l'on sait que la cinétique de diffusion est d'ordre 0 et qu'au moins 80% du composé d'iode sera libéré suivant cette cinétique.

De façon à maîtriser la libération du principe actif, il est avantageux de présenter la matrice silicone sous la forme de modules (éléments) élémentaires de formes variées tels que des cubes, des parallélépipèdes rectangles, des cylindres, des sphères dont les paramètres fondamentaux sont les suivants :

— la nature du composé d'iode,

— le diamètre moyen (granulométrie) g des particules du composé d'iode dans le cas préféré où ce dernier est un solide,

— la teneur t de composé d'iode au sein de la matrice,

— le rapport R de la surface au volume du module.

La nature du composé d'iode et sa granulométrie définissent la vitesse de diffusion à travers la matrice, du principe actif.

Plus g est petit et plus v est lent et inversement.

Plus t est grand et plus le flux de principe actif est élevé et inversement.

Plus R est grand et plus le flux élevé de principe actif est grand et inversement.

L'homme de métier, par des expériences de routine, peut sans difficulté aboutir rapidement au résultat visé en extrapolant le temps d'élution théorique qui correspondra au temps réel de diffusion du principe actif.

Pour NaI et $KIO_3$ qui sont les composés d'iode préférés, g, t et R peuvent être avantageusement choisis dans les zones suivantes :

— g compris entre 1 et 300 μm,

— t compris entre 10 et 100 parties en poids de composé d'iode pour 100 parties de (A),

— R compris entre 0,5 et 50 pour une forme cylindrique.

Le composé diode est dispersé de façon homogène au sein de la matrice.

Les huiles diorganopolysiloxanes (A) utilisables dans les compositions selon l'invention sont plus particulièrement celles répondant à la formule (1) :

$$Y_nSi_{3-n}O(SiR_2O)_xSiR_{3-n}Y_n \quad (1)$$

dans laquelle :

R représente des radicaux hydrocarbonés monovalents identiques ou différents, Y représente des groupes hydrolysables ou condensables identiques ou différents, ou des groupes hydroxy, n est choisi parmi 2 et 3 quand Y représente un groupe hydrolysable ou condensable et est égal à 1, quand Y est un hydroxy, et x est un nombre entier supérieur à 1, de préférence supérieur à 10.

La viscosité des huiles de formule (1) est comprise entre 50 et $10^6$ mPa · s à 25°C. Comme exemple de radicaux R on peut citer les radicaux alkyle ayant de 1 à 8 atomes de carbone tels que méthyle, éthyle, propyle, butyle, héxyle et octyle, les radicaux vinyle, les radicaux phényle. Comme exemples de radicaux R substitués on peut citer les radicaux trifluoro-3, 3, 3 propyle, chlorophényle et bétacyanoéthyle.

Dans les produits de formule (1) généralement utilisés industriellement, au moins 60% en nombre des radicaux R sont des radicaux méthyle, les autres radicaux étant généralement des radicaux phényle et/ou vinyle.

Comme exemple de groupe Y hydrolysables on peut citer les groupes amino, acylamino, aminoxy, cétiminoxy, iminoxy, énoxy, alcoxy, alcoxy-alkylène-oxy, acyloxy et phosphato.

Comme exemples de groupes Y amino, on peut citer les groupes n-butylamino, sec-butylamino et cyclohexylamino, de groupes acylamino N substitué, on peut citer le groupe benzoyl-amino, de groupes aminoxy les groupes diméthylaminoxy, diéthylaminoxy, dioctylaminoxy et diphénylaminoxy, comme exemples de groupes Y iminoxy et cétiminoxy, ceux dérivés de l'acétophénone-oxime, l'acétone-oxime, la benzophènone-oxime, la méthyl-éthyl cétoxime, la diisopropylcétoxime et la chlorocyclo-hexanone-oxime.

Comme groupes Y alcoxy on peut citer les groupes ayant de 1 à 8 atomes de carbone comme les groupes méthoxy, éthoxy, propoxy, isopropoxy, butoxy, hexyloxy et octyloxly, comme groupe Y alcoxy-alkylène-oxy on peut citer le groupe méthoxy-éthylène-oxy.

Comme groupes Y alcyloxy, on peut citer les groupes ayant de 1 à 8 atomes de carbone comme les groupes formyloxy, acétoxy, propionyloxy et éthyl-2 hexanoyloxy.

Comme groupes Y phospato on peut citer ceux dérivant des groupes phosphate de diméthyle, phosphate de diéthyle et phosphate de dibutyle.

Comme groupes Y condensables on peut citer les atomes d'hydrogène et les atomes d'halogène, de préférence le chlore.

Quand dans la formule (1) ci-dessus les groupes Y sont des groupes hydroxy, n est alors égal à 1, il est nécessaire, pour préparer des élastomères polyorganosiloxanes à partir des polymères de formule (1) ci-des-

5

sus, d'utiliser, en plus des catalyseurs de condensation, des agents réticulants (D) déjà indiqués qui sont des silanes de formule générale :

$$R_{4-a}SiY'_a \quad (2)$$

dans laquelle R a les significations données plus haut à la formule (1) et Y' représente des groupes hydrolysables ou condensables, identiques ou différents, a est égal à 3 ou 4.

Les exemples donnés pour les groupes Y sont appliquables aux groupes Y'.

Il est souhaitable d'utiliser des silanes de formule (2) même dans le cas où dans l'huile (A) Y n'est pas un groupe hydroxy.

Dans ce cas il est souhaitable d'utiliser des groupes Y de l'huile (A) identiques aux Y' du silane (D).

Les diorganopolysiloxanes alpha-oméga dihydroxylés de formule (1) sont généralement des huiles dont la viscosité varie de 500 mPa · s à 25°C à 500000 mPa · s à 25°C, de préférence 800 mPa · s à 400000 à 25°C, ce sont des polymères linéaires constitués essentiellement de motifs diorganosiloxyles de formule ($R_2SiO$).

Toutefois la présence d'autres motifs, présents généralement à titre d'impuretés, tels que $R\,SiO_{3/2}$, $R\,SiO_{1/2}$ et $SiO_{4/2}$ n'est pas exclue dans la proportion d'au plus 1% par rapport au nombre de motifs diorganosiloxyles.

Les radicaux organiques, liés aux atomes de silicium des huiles de base, représentés par le symbole R, peuvent être choisis parmi les radicaux alkyles ayant de 1 à 3 atomes de carbone tels que les radicaux méthyle, éthyle, n-propyle, le radical vinyl, le radical phényle, le radical trifluoro-3, 3, 3 propyle et le radical bétacyanoétyle.

Au moins 60% de l'ensemble des radicaux R sont des radicaux méthyle, au plus 1% sont des radicaux vinyle.

A titre illustratif de motifs représentés par la formule $R_2SiO$ peuvent être cités ceux de formules :

$(CH_3)_2SiO$ ;
$CH_3(CH_2=CH)SiO$ ;
$CH_3(C_6H_5)SiO$ ;
$CF_3CH_2CH_2(CH_3)SiO$ ;
$NC-CH_2CH_2(CH_3)SiO$ ;
$NC-CH_2(C_6H_5)SiO$ ;

Ces huiles de base sont, dans leur grande majorité, commercialisées par les fabricants de silicones. D'autre part leurs techniques de fabrication sont bien connues, on les trouve décrites par exemple dans les brevets français FR-A-1134005, FR-A-1198749, FR-A-1226745.

Comme exemples de silanes monomères (D) de formule (2) on peut citer plus particulièrement les polyacyloxysilanes, les polyalcoxysilanes, les polycétiminoxysilanes et les polyiminoxysilanes et en particulier les silanes suivants :

$CH_3Si(OCOCH_3)_3$ ;
$C_2H_5Si(OCOCH_3)_3$ ;
$CH_2 = CHSi(OCOCH_3)_3$ ;
$C_6H_5Si(OCOCH_3)_3$ ;
$CF_3CH_2CH_2Si(OCOCH_3)_3$ ;
$NC-CH_2CH_2Si(OCOCH_3)_3$ ;
$CH_2ClSi(OCOCH_3)_3$ ;
$CH_3Si(ON=C(CH_3)C_2C_5)_2OCH_2CH_2OCH_3$ ;
$CH_3Si(ON=CH-CH_3)_2OCH_2CH_2OCH_3$.

Les silanes (D) ci-dessus associés à des polydiorganosiloxanes alpha-oméga dihydroxylés de formule (1) peuvent être utilisés en compositions monocomposantes stables à l'abri de l'air.

Comme exemples de silane monomère de formule (2) qui, associés à des polydiroganosiloxanes alpha-oméga dihydroxylés de formule (1), peuvent être utilisés avantageusement en compositions bi-composantes, on peut citer les polyalcoxysilanes et en particulier ceux de formules :

$Si(OC_2H_5)_4$ ;
$Si(O-n-C_3H_7)_4$ ;
$Si(O-isoC_3H_7)_4$ ;
$Si(OC_2H_4OCH_3)_4$ ;
$CH_3Si(OCH_3)_3$ ;
$CH_2 = CHSi(OCH_3)_3$ ;
$CH_3Si(OC_2H_4OCH_3)_3$ ;
$ClCH_2Si(OC_2H_5)_3$ ;
$CH_2 = CHSi(OC_2H_4OCH_3)_3$.

A tout ou partie des silanes monomères ci-dessus décrits on peut substituer des polyalcoxypolysiloxanes dont chaque molécule compte au moins deux, de préférence trois atomes Y', les autres valences du silicium sont satisfaites par des liaisons siloxaniques SiO- et SiR. Comme exemples d'agents réticulants polymères on peut citer le polysilicate d'éthyle.

On utilise généralement de 0,1 à 20 parties en poids d'agent réticulant de formule (2) pour 100 parties en poids de polymère de formule (1).

Les compositions polyorganosiloxanes durcissables en élastomère du type décrit ci-dessus comportent de 0,001 à 10 parties en poids, de préférence de 0,05 à 3 parties en poids de catalyseur de condensation (C) pour 100 parties en poids de polysiloxane de formule (1).

La teneur en catalyseur de condensation des compositions mono-composantes est généralement beaucoup plus faible que celle utilisée dans les compositions bi-composantes et est généralement comprise entre 0,001 et 0,05 parties en poids pour 100 parties en poids de polysiloxane de formule (2).

Les agents réticulants (D) de formule (2), qu'ils soient utilisables pour la préparation des compositions monocomposantes ou bi-composantes, sont des produits accessibles sur le marché des silicones de plus leur emploi dans les compositions durcissant dès la température ambiante est connu, il figure notamment dans les brevets français FR-A-1126411, FR-A-1179969, FR-A-1189216, FR-A-1198749, FR-A-1248826, FR-A-1314649, FR-A-1423477, FR-A-1432799 et FR-A-2067636.

Les compositions selon l'invention peuvent comporter en outre des charges (E) renforçantes ou semi-renforçantes ou de bourrage qui sont de préférence choisies parmi les charges siliceuses, les silices de combustion et les silices de précipitation. Elles ont une surface spécifique, mesurée selon les méthodes BET, d'au moins 50 m²/g, de préférence supérieure à 70 m²/g, une dimension moyenne des particules primaires inférieure à 0,1 μm (micromètre) et une densité apparente inférieure à 200 g/litre.

Ces silices peuvent être incorporées de préférence telles quelles ou après avoir été traitées par des composés organosiliciques habituellement utilisés pour cet usage. Parmi ces composés, figurent les méthylpolysiloxanes tels que l'hexaméthyldisiloxane, l'octaméthylcyclotétrasiloxane, des méthylpolysilazanes tels que l'hexaméthyldisilazane, l'hexaméthylcyclotrisilazane, des chlorosilanes tels que le diméthyldichlorosilane, le triméthylchlorosilane, le méthyvinyldichlorosilane, le diméthylvinylchlorosilane, des alcoxysilanes tels que le diméthyldiméthoxysilane, le diméthylvinyléthoxysilane, le triméthylméthoxysilane. Lors de ce traitement, les silices peuvent accroître leur poids de départ jusqu'à un taux de 20%, de préférence 18% environ.

Les charges semi-renforçantes ou de bourrage ont un diamètre particulaire supérieur à 0,1 μm et sont choisies parmi le quartz broyé, les argiles calcinées et les terres de diatomées.

On peut généralement utiliser de 0 à 100 parties, de préférence de 5 à 50 parties de charge (E) pour 100 parties d'huile (A).

Les bases de compositions de silicone définies de façon générale ci-dessus sont bien connues de l'homme de métier. Elles sont décrites en détail dans la littérature en particulier dans de nombreux brevets et la plupart sont disponibles dans le commerce.

Ces compositions réticulent à température ambiante en présence d'humidité apportée par l'humidité de l'air et/ou contenue dans la composition. Elles se divisent en deux grandes familles. La première famille est constituée par les compositions mono-composantes ou à un seul emballage stables au stockage à l'abri de l'humidité de l'air et durcissent en élastomère à l'humidité de l'air. Dans ce cas le catalyseur de condensation (C) utilisé est un composé métallique généralement un composé de l'étain, du titane ou du zirconium.

Selon la nature des groupements condensables ou hydrolysables ces compositions mono-composantes sont dites acides, neutres, ou basiques.

Comme compositions acides on peut utiliser par exemple les compositions décrites dans les brevets US-A-3035016, US-A-3077465, US-A-3133891, US-A-3409573, US-A-3438930, US-A-3647917 et US-A-3886118.

Comme compositions neutres, on peut utiliser par exemple les compositions décrites dans les brevets US-A-3065194, US-A-3542901, US-A-3689454, US-A-3779986, GB-A-2052540, US-A-4417042 et EP-A-69256.

Comme compositions basiques on peut par exemple utiliser les compositions décrites dans les brevets US-A-3378520, US-A-3364160, US-A-3417047, US-A-3464951, US-A-3742004 et US-A-3758441.

On peut également utiliser selon une variante préférée les compositions coulantes mono-composantes telles que celles décrites dans les brevets US-A-3922246, US-A-3965280 et US-A-4143088.

La deuxième famille qui est la famille préférée dans le cadre de la présente invention est constituée par les compositions à deux composantes ou à deux emballages comportant généralement une huile (A) alphaoméga-dihydroxydiorganopolysiloxane, un silane (D) ou un produit provenant de l'hydrolyse partielle de ce silane, et un catalyseur (C) qui est un composé métallique de préférence un composé de l'étain et/ou une amine.

Des exemples de telles compositions sont décrits dans les brevets US-A-3678002, US-A-3888815, US-A-3933729, US-A-4064096 et GB-A-2032936.

Parmi ces compositions on préfère plus particulièrement les compositions bi-composantes comportant :

(A) : 100 parties d'une huile alpha-oméga-dihydroxyorganopolysiloxane de viscosité de 50 à 300000 mPa·s dont les radicaux organiques sont choisis parmi les radicaux méthyle, éthyle, vinyle, phényle et trifluoro-3, 3, 3 propyle, au moins 60% en nombre étant des radicaux méthyle, jusqu'à 20% en nombre étant des radicaux phényle, au plus 2% étant des radicaux vinyle,

(B) : 10 à 130 parties d'un composé d'iode,

(C) : 0,01 à 1 partie (calculée en poids d'étain métal) d'un composé catalytique de l'étain,

(D) : 0,5 à 15 parties d'un polyalcoxysilane ou un polyalcoxysiloxane,

(E) : 0 à 100 parties, de préférence 5 à 50 parties de charge minérale siliceuse.

Les catalyseurs (C) à l'étain sont abondamment décrits dans la littérature citée ci-dessus, ce peut être en particulier un sel d'étain d'un acide mono- ou dicarboxylique. Ces carboxylates d'étain sont décrits notamment dans l'ouvrage de NOLL (Chemistry and Technology of Silicones, page 337, Academic Press, 1968, 2ème édition). On peut on particulier citer le napthénate, l'octanoate, l'oléate, le butyrate, le dilaurate de dibutylétain, le diacétate de dibutylétain.

On peut également utiliser comme composé catalytique à l'étain le produit de réaction d'un sel d'étain, en particulier d'un dicarboxylate d'étain sur du polysilicate d'éthyle comme décrit dans le brevet US-A-3186963. On peut également utiliser le produit de réaction d'un dialkyldialcoxysilane sur un carboxylate d'étain comme décrit dans le brevet US-A-3862919.

On peut également utiliser le produit de réaction d'un silicate d'alkyle ou d'un alkyltrialcoxysilane sur le diacétate de dibutylétain comme décrit dans le brevet belge BE-A-842305.

Parmi les agents de réticulation (S) on préfère plus particulièrement les alkyltrialcoxysilanes, les silicates d'alkyle et les polysilicates d'alkyle dans lesquels les radicaux organiques sont des radicaux alkyles ayant de 1 à 4 atomes de carbone.

Les silicates d'alkyle peuvent être choisis parmi le silicate de méthyle, le silicate d'éthyle, le silicate d'isopropyle, le silicate de n-propyle, et les polysilicates choisis parmi les produits d'hydrolyse partielle de ces silicates ; ce sont des polymères constitués d'une proportion importante de motifs de formule $(R^4O)_3SiO_{0,5}$, $R^4OSiO_{1,5}$, $(R^4O)_2SiO$ et $SiO_2$ ; le symbole $R^4$ représentant les radicaux méthyle, éthyle, isopropyle, n-propyle. Pour les caractériser on se base habituellement sur leur teneur en silice qui est établie par dosage du produit d'hydrolyse totale d'un échantillon.

On peut en particulier utiliser comme polysilicate, un silicate d'éthyle partiellement hydrolysé commercialisé sous la marque "Ethyl Silicate-40R par Union Carbide Corporation, ou un silicate de propyle partiellement hydrolysé.

Les compositions selon l'invention peuvent être extrudées ou moulées suivant des formes variées, par exemple sous forme de modules (éléments) unitaires, puis à être durcies à température ambiante en un élastomère à l'humidité atmosphérique ou par addition d'eau. Un léger chauffage à une température de 20 à 150°C peut accélérer le durcissement.

Les modules (éléments) de formes variées en élastomère peuvent être tenues immergées dans les eaux à traiter suivant une quantité (un poids d'élastomère) telle que l'élastomère assure une libération continue et contrôlée d'iode de préférence pendant au moins un an. Au bout de cette période les modules (éléments) sont remplacés.

De façon surprenante on a découvert que ces compositions de silicones réticulées présentent des caractéristiques physique suffisantes pour les applications envisagées.

Les exemples suivants illustrent l'invention sans en limiter la portée.

Exemple 1.

On prépare un empâtage par malaxage de :
* 75 parties d'huile alpha oméga-dihydroxyméthylpolysiloxane de viscosité 15.000 mPa · s à 25°C.
* 22,2 parties de célite (terre de diatomées).
* 2,3 parties de silicate de n-propyle.
* 0,5 partie d'un empâtage hydraté (mélange d'huile diméthyl polysiloxane et de silice de combustion) contenant 5% d'eau.

Le mélange est homogénéisé pendant 4 heures puis filtré.

A 100 parties de cet empâtage on ajoute 25 parties de NaI de granulométrie moyenne égale à 5 µm.

On obtient un produit coulant que l'on catalyse par introduction de 0,5 g d'éthyl-2 hexanoate d'étain.

Le produit est coulé dans des moules en acier recouvert d'un agent antiadhérent (Mélinex), de forme cylindrique (diamètre égal à 10 mm).

On obtient au bout de 1 heure à 100°C un produit bien réticulé et ayant les propriétés mécaniques suffisantes pour l'application envisagée.

* Protocole expérimental de la mesure de la cinétique d'élution.

La composition élastomère contenant le NaI est découpé à longueur désirée (20 mm), conformément au rapport Surface/Volume (4,80 cm⁻¹) que l'on désire atteindre et immergée dans un récipient 600 ml d'eau distilée, thermostatée à 20°C.

Le récipient est équipé d'un système d'agitation magnétique, animé d'un mouvement de rotation lent (100 tr/min) assurant l'homogénéité de la solution. Il est recouvert d'un couvercle afin de minimiser l'évaporation de l'eau.

Des prélévements journaliers de 1 ml sont réalisés dans les premiers temps de l'élution, et hebdomadaires au bout de 15 jours d'élution.

La concentration en iodure ou iodate, libérée par jour, est déterminée par un dosage avec une électrode spécifique à iodure :

A un millilitre de prélévement du récipient on rajoute deux millilitres d'une solution (K2SO4 + acide ascorbique) — cette solution joue le rôle de tampon ionique, et de solution réductrice dans le cas du dosage des iodates — et un millilitre d'eau distillée. On immerge l'électrode dans cette solution et on lit le potentiel électrochimique de la solution. Une courbe d'étalonnage préalablement établie avec des solutions en iodure de 5.10-5 M/l (M : mole) à 5.10-2 M/l permet de calculer la concentration (C) en iodure ou iodate en mg/l de la solution.

Les caractéristiques du cylindre immergé sont :
- Diamètre = 10 mm
- Hauteur = 20 mm
- Surface = 8,3 cm²
- Volume = 1,72 cm³
- S/V = 4,80 cm⁻¹
- Masse totale = 2,04 g
- Quantité initiale de I (Qo) = 0,242 g

Dans le tableau suivant (Tableau 1) sont rassemblés les résultats de la cinétique d'élution.

| Temps (jours) | Q cumulé (gramme) ION ACTIF | 100 x Q/Qo % |
|---------------|------------------------------|--------------|
| 0.25          | 0,003                        | 1.32         |
| 0.92          | 0,007                        | 2,62         |
| 1.92          | 0,009                        | 3,11         |
| 2,25          | 0,010                        | 3,28         |
| 5,00          | 0,011                        | 3,75         |
| 12,00         | 0,014                        | 4,84         |
| 22,00         | 0,018                        | 5,97         |
| 36,00         | 0,027                        | 9,06         |
| 50,00         | 0,036                        | 12,04        |

Tableau 1.

Q cumulé correspond à la quantité d'équivalent I (que nous appelons ion actif) éluée à l'instant t.

Sachant que 80% de l'ion actif incorporé élue selon une cinétique d'ordre zéro en fonction du temps, nous pouvons calculer le temps d'élution théorique de l'expérience avec l'expression suivante :

$$Te = \frac{0,8 \times Qo}{\text{Flux journalier.}} \quad (\text{jours})$$

Pour l'exemple 1, le temps d'élution théorique est de 333 jours.

Exemple 2

On procède exactement comme à l'exemple 1, sauf que l'on remplace NaI par 25 parties de KIO3 de granulométrie comprise entre 100 et 200 µm.

On immerge dans 600 ml d'eau distillée, thermostatée à 20°C, un cylindre dont les caractéristiques sont les suivantes :

- Diamètre = 10 mm
- Hauteur = 20 mm
- Surface = 8,3 cm$^2$
- Volume = 1,72 cm$^3$
- S/V = 4,80 cm$^{-1}$
- Masse totale = 2,04 g
- Quantité initiale de I (Qo) = 0,169 g

Le protocole expérimental de la mesure de la cinétique d'élution est identique à celui de l'exemple 1. Les résultats sont rassemblés dans le tableau 2, ci-après.

| Temps (jours) | Q cumulé (gramme) ION ACTIF | 100 x Q/Qo % |
|---|---|---|
| 0.92 | 0.004 | 2.50 |
| 1.92 | 0.005 | 3.07 |
| 2.25 | 0.005 | 3.16 |
| 5.00 | 0.006 | 3.90 |
| 12,00 | 0,008 | 4.80 |
| 22,00 | 0,010 | 6.20 |
| 36,00 | 0.014 | 8.78 |
| 50,00 | 0,019 | 11.32 |

Tableau 2

Le temps d'élution théorique pour cet exemple est de 353 jours.

## Revendications

1. Composition diorganopolysiloxane durcissable en un élastomère de silicone par des réactions de polycondensation, caractérisée en ce qu'il comporte :

(A) : au moins une huile diorganopolysiloxane portant à chaque extrémité de la chaîne au moins deux groupes condensables ou hydrolysables, ou un seul groupe hydroxy, ladite huile ayant une viscosité à 25°C inférieure à 1000000 mPa · s,

(B) : au moins un composé de l'iode, à l'exclusion de l'iode moléculaire $I_2$, solide à température ambiante, soluble dans l'eau, non toxique dispersé de façon homogène dans la composition et n'inhibant pas le durcissement de la composition en un élastomère,

(C) : un catalyseur de polycondensation de l'huile,

(D) : un silane comportant au moins trois groupes condensables ou hydrolysables, notamment quand (A) est une huile à extrémités hydroxy.

2. Composition selon la revendication 1, caractérisée en ce que pour 100 parties, en poids, d'huile (A) elle comprend 5 à 130 parties de composé (B).

3. Composition de silicone selon la revendication 1, caractérisée en ce que le composé de l'iode (B) est un iodure ou iodate de formules générales :

$$(M^{a+})\ (I^-)_a$$
et $\quad (M^{a+})\ (IO_3^-)_a$

dans lesquelles a est un nombre entier supérieur ou égal à 1 et M est un cation choisi parmi un métal alcalin, un métal alcalino-terreux, un métal de transition et un ammonium quaternaire $(NZ_4)^+$ dont les radicaux Z identiques ou différents représentent un radical alkyle linéaire ou ramifié en $C_1$-$C_{20}$ ou un atome d'hydrogène.

4. Composition selon la revendication 1, caractérisée en ce que le composé de l'iode (B) est choisi parmi :
Na I, Na $IO_3$,
K I, K $IO_3$,
Mg $I_2$, Mg $I_2 \cdot 8H_2O$,
$Mg(IO_3)_2 \cdot 4H_2O$,
$NH_4$ I
Fe $I_2 \cdot 4H_2O$
Mn $I_2$

5. Composition selon l'une des revendications 3 ou 4, caractérisée en ce qu'elle comporte de 5 à 130 parties en poids de composé de l'iode (B) pour 100 parties en poids de l'huile (A).

6. Composition selon l'une quelconque des revendications précédentes, caractérisée en ce que le diorganopolysiloxane (A) répond à la formule générale (1) :

$$Y_nSi_{3-n}O(SiR_2O)_xSiR_{3-n}Y_n \quad (1)$$

dans laquelle :
R représente des radicaux hydrocarbonés monovalents identiques ou différents, Y représente des groupes hydrolysables ou condensables identiques ou différents, ou des groupes hydroxy,
avec n = 1 quand Y est un hydroxy, et avec n = 2 ou 3 quand Y représente un groupe hydrolysable, et x est un nombre entier supérieur à 1, de préférence supérieur à 10.

7. Composition selon la revendication 6, caractérisée en ce que le radical R est choisi parmi les radicaux alkyle en $C_1$-$C_8$, vinyle, phényle et trifluoro-3, 3, 3 propyle, au moins 60% en nombre des radicaux R étant des radicaux méthyle.

8. Composition selon la revendication 6 ou 7, caractérisée en ce que Y est choisi parmi les groupes amino, acylamino, aminoxy, cétiminoxy, iminoxy, énoxy, alcoxy, alcoxy-alkylènoxy, acyloxy et phosphato.

9. Composition selon l'une quelconque des revendications précédentes, caractérisée en ce qu'elle comporte un silane (D) dans le cas où les groupes condensables ou hydrolysables ne sont pas des groupes hydroxy et en ce que le silane (D) répond à la formule :

$$R_{4-a}SiY'_a \quad (2)$$

dans laquelle R est un radical hydrocarboné monovalent, Y' représente des groupes hydrolysables ou condensables, identiques ou différents, et a est égal à 3 ou 4.

10. Composition selon l'une quelconque des revendications précédentes, caractérisée en ce qu'elle est monocomposante.

11. Composition selon l'une quelconque des revendications 1 à 10, caractérisée en ce qu'elle est bi-com-

EP 0 283 408 B1

posante.

12. Composition selon la revendication 11, caractérisée en ce qu'elle comporte

(A) : 100 parties en poids d'une huile alpha-oméga-dihydroxyorganopolysiloxane de viscosité de 50 à 300000 mPa · s dont les radicaux organiques sont choisis parmi les radicaux méthyle, éthyle, vinyle, phényle et trifluoro-3, 3, 3 propyle, au moins 60% en nombre étant des radicaux méthyle, jusqu'à 20% en nombre étant des radicaux phényle, au plus 2% étant des radicaux vinyle,

(B) : 10 à 130 parties en poids du composé de l'iode,

(C) : 0,01 à 1 partie en poids (calculée en poids d'étain métal) d'un composé catalytique de l'étain,

(D) : 0,5 à 15 parties en poids d'un polyalcoxysilane ou un polyalcoxysiloxane,

(E) : 0 à 100 parties, de préférence 5 à 50 parties en poids de charge minérale siliceuse.

13. Composition selon l'une quelconque des revendications précédentes, durcie en un élastomère.

14. Procédé de traitement d'eau, caractérisé en ce qu'on immerge une quantité adaptée d'un élastomère telle que définie à la revendication 13 en vue de libérer dans l'eau une quantité continue et contrôlée d'iode.

**Patentansprüche**

1. Zu einem Siliconelastomeren durch Polykondensationsreaktionen härtbare Diorganopolysiloxanzusammensetzung, dadurch gekennzeichnet, daß sie enthält :

(A) zumindest ein Diorganopolysiloxanöl, das an jedem Kettenende zumindest zwei kondensierbare oder hydrolysierbare Gruppen oder eine einzige Hydroxylgruppe enthält, wobei das Öl bei 25°C eine Viskosität von geringer als 1000000 Pa · s besitzt,

(B) zumindest eine Jodverbindung unter Ausschluß von molekularem Jod $J_2$, die bei Raumtemperatur fest, in Wasser löslich, nicht-toxisch, in der Zusammensetzung homogen dispergiert und die Härtung der Zusammensetzung zu einem Elastomeren nicht inhibierend ist,

(C) einen Polykondensationskatalysator des Öls,

(D) ein Silan mit zumindest drei kondensierbaren oder hydrolysierbaren Gruppen, insbesondere wenn (A) ein Öl mit Hydroxyendgruppen ist.

2. Zusammensetzung gemäß Anspruch 1, dadurch gekennzeichnet, daß sie je 100 Gew.Teile Öl (A) 5 bis 130 Teile Verbindung (B) enthält.

3. Siliconzusammensetzung gemäß Anspruch 1, dadurch gekennzeichnet, daß die Jodverbindung (B) ein Jodid oder Jodat der allgemeinen Formeln

$(M^{a+}) (J^-)_a$

und $(M^{a+}) (JO_3^-)_a$

ist, worin a eine ganze Zahl von größer als oder gleich 1 ist und M für ein Kation steht, ausgewählt unter einem Alkalimetall, einem Erdalkalimetall, einem Übergangsmetall und einem quaternären Ammonium $(NZ_4)^+$, dessen Reste Z, die gleich oder verschieden sind, einen linearen oder verzweigten $C_{1-20}$-Alkylrest oder ein Wasserstoffatom bedeuten.

4. Zusammensetzung gemäß Anspruch 1, dadurch gekennzeichnet, daß die Jodverbindung (B) ausgewählt ist unter

Na J, Na $JO_3$,

K J, K $JO_3$,

Mg $J_2$, Mg $J_2 \cdot 8H_2O$,

Mg$(JO_3)_2 \cdot 4H_2O$,

$NH_4$ J

Fe $J_2 \cdot 4H_2O$

Mn $J_2$.

5. Zusammensetzung gemäß einem der Ansprüche 3 oder 4, dadurch gekennzeichnet, daß sie 5 bis 130 Gew.Teile der Jodverbindung (B) je 100 Gew.Teile Öl (A) enthält.

6. Zusammensetzung gemäß einem der vorhergehenden Ansprüche, dadurch gekennzeichnet, daß das Diorganopolysiloxan (A) der allgemeinen Formel (1)

12

$$Y_n Si_{3-n} O(SiR_2 O)_x SiR_{3-n} Y_n \quad (1)$$

entspricht, worin

R gleiche oder verschiedene, einwertige Kohlenwasserstoffreste bedeutet, Y gleiche oder verschiedene hydrolysierbare oder kondensierbare Gruppen oder Hydroxygruppen darstellt,

mit n = 1, wenn Y für eine hydroxygruppe steht, und mit n = 2 oder 3, wenn Y für eine hydrolysierbare Gruppe steht, und x eine ganze Zahl von größer als 1 und vorzugsweise größer als 10 ist.

7. Zusammensetzung gemäß Anspruch 6, dadurch gekennzeichet, daß der Rest R ausgewählt ist unter den $C_{1-8}$-Alkylresten, Vinyl, Phenyl und 3, 3, 3-Trifluorpropyl, wobei zumindest 60% der Anzahl der Reste R Methylreste sind.

8. Zusammensetzung gemäß Anspruch 6 oder 7, dadurch gekennzeichnet, daß Y ausgewählt ist unter den Amino-, Acylamino-, Aminoxy-, Ketiminoxy-, Iminoxy-, Enoxy-, Alkoxy-, Alkoxy-alkylenoxy-, Acyloxy- und Phosphato-gruppen.

9. Zusammensetzung gemäß einem der vorhergehenden Ansprüche, dadurch gekennzeichnet, daß sie ein Silan (D) enthält, wenn die kondensierbaren oder hydrolysierbaren Gruppen keine Hydroxygruppen sind, und daß das Silan (D) der Formel

$$R_{4-a} SiY'_a \quad (2)$$

entspricht, worin R einen einwertigen Kohlenwasserstoffrest bedeutet, Y' für hydrolysierbare oder kondensierbare Gruppen, die gleich oder verschieden sind, steht und a 3 oder 4 ist.

10. Zusammensetzung gemäß einem der vorhergehenden Ansprüche, dadurch gekennzeichnet, daß sie eine Ein-Komponenten-Zusammensetzung ist.

11. Zusammensetzung gemäß einem der Ansprüche 1 bis 10, dadurch gekennzeichnet, daß sie eine Zwei-Komponenten-Zusammensetzung ist.

12. Zusammensetzung gemäß Anspruch 11, dadurch gekennzeichnet, daß sie enthält

(A)     100 Gew.Teile eines $\alpha$, $\omega$ -Dihydroxyorganopolysiloxanöls mit einer Viskosität von 50 bis 300000 mPa·s, dessen organische Reste unter den Methyl-, Ethyl-, Vinyl-, Phenyl- und 3, 3, 3-Trifluorpropylresten ausgewählt sind, wobei zumindest 60%, bezogen auf die Anzahl, Methylreste sind, bis zu 20%, bezogen auf die Anzahl, Phenylreste sind und höchstens 2% Vinylreste sind ;

(B)     10 bis 130 Gew.Teile Jodverbindung ;

(C)     0,01 bis 1 Gew.Teil (berechnet auf das Gewicht des Zinnmetalls bezogen) einer katalytischen Zinnverbindung ;

(D)     0,5 bis 15 Gew.Teile eines Polyalkoxysilans oder eines Polyalkoxysilans ;

(E)     0 bis 100 Teile, vorzugsweise 5 bis 50 Teile, auf das Gewicht bezogen, an siliciumhaltigen, mineralischen Füllstoffen.

13. Zusammensetzung gemäß einem der vorhergehenden Ansprüche, welche zu einem Elastomeren gehärtet ist.

14. Verfahren zur Behandlung von Wasser, dadurch gekennzeichnet, daß man eine Menge eines Elastomeren, wie in Anspruch 13 definiert, die hinsichtlich der Freisetzung einer kontinuierlichen und kontrollierten Jodmenge in Wasser geeignet ist, eintaucht.

## Claims

1. Diorganopolysiloxane composition curable to form a silicone elastomer by polycondensation reactions, characterised in that it comprises :

(A) :     at least one diorganopolysiloxane oil carrying at each end of the chain at least two condensable or hydrolysable groups, or a single hydroxyl group, the said oil having a viscosity at 25°C of less than 1,000,000 mPa · s,

(B) :     at least one iodine compound, with the exclusion of molecular iodine $I_2$, which is solid at ambient temperature, soluble in water, nontoxic and homogeneously dispersed in the composition and which does not inhibit the curing of the composition to form an elastomer,

(C) :     a polycondensation catalyst for the oil, and

(D) :     a silane comprising at least three condensable or hydrolysable groups, in particular when (A) is an

EP 0 283 408 B1

oil having hydroxyl ends.

2. Composition according to Claim 1, characterised in that it comprises 5 to 130 parts of compound (B) per 100 parts, by weight, of oil (A).

3. Silicon composition according to Claim 1, characterised in that the iodine compound (B) is an iodide or iodate of general formulae :

$$(M^{a+}) (I^-)_a$$
and $$(M^{a+}) (IO_3^-)_a$$

in which formulae a is an integer greater than or equal to 1 and M is a cation chosen from an alkali metal, an alkaline earth metal, a transition metal and a quaternary ammonium $(NZ_4)^+$, in which the radicals Z, which may be identical or different, represent a $C_1$-$C_{20}$ straight-chain or branched alkyl radical or a hydrogen atom.

4. Composition according to Claim 1, characterised in that the iodine compound (B) is chosen from :

Na I, Na $IO_3$,

K I, K $IO_3$

Mg $I_2$, Mg $I_2 \cdot 8H_2O$,

Mg $(IO_3)_2 \cdot 4H_2O$,

$NH_4$ I

Fe $I_2 \cdot 4H_2O$

Mn $I_2$

5. Composition according to one of Claims 3 or 4, characterised in that it comprises from 5 to 130 parts by weight of iodine compound (B) per 100 parts by weight of the oil (A).

6. Composition according to any one of the preceding claims, characterised in that diorganopolysiloxane (A) corresponds to the general formula (1) :

$$Y_nSi_{3-n}O(SiR_2O)_xSiR_{3-n}Y_n \quad (1)$$

in which :

R represents identical or different monovalent hydrocarbon radicals, Y represents identical or different hydrolysable or condensable groups, or hydroxyl groups, where n = 1 when Y is a hydroxyl, and where n = 2 or 3 when Y represents a hydrolysable group, and x is an integer greater than 1 and preferably greater than 10.

7. Composition according to Claim 6, characterised in that the radical R is chosen from $C_1$-$C_8$alkyl, vinyl, phenyl and 3, 3, 3-trifluoropropyl radicals, at least 60% by number of the radicals R being methyl radicals.

8. Composition according to Claim 6 or 7, characterised in that Y is chosen from amino, acylamino, aminoxy, ketiminoxy, iminoxy, enoxy, alkoxy, alkoxyalkyleneoxy, acyloxy and phosphato groups.

9. Composition according to any one of the preceding claims, characterised in that it comprises a silane (D) in the case where the condensable or hydrolysable groups are not hydroxyl groups and in that the silane (D) corresponds to the formula :

$$R_{4-a}SiY'_a \quad (2)$$

in which R is a monovalent hydrocarbon radical, Y' represents identical or different hydrolysable or condensable groups and a is 3 or 4.

10. Composition according to any one of the preceding claims, characterised in that it is a one-component composition.

11. Composition according to any one of Claims 1 to 9, characterised in that it is a two-component composition.

12. Composition according to Claim 11, characterised in that it comprises

(A) : 100 parts by weight of an alpha,omega-dihydroxyorganopolysiloxane oil having a viscosity of 50 to 300,000 Pa · s, the organic radicals of which are chosen from methyl, ethyl, vinyl, phenyl and 3, 3, 3-trifluoropropyl radicals, at least 60% by number being methyl radicals, up to 20% by number being phenyl radicals and at most 2% being vinyl radicals,

(B) : 10 to 130 parts by weight of the iodine compound,

(C) : 0.01 to 1 part by weight (calculated as weight of tin metal) of a catalytic tin compound,

(D) : 0.5 to 15 parts by weight of a polyalkoxysilane or a polyalkoxysiloxane, and

(E) : 0 to 100 parts, preferably 5 to 50 parts, by weight of siliceous inorganic filler.

14

13. Composition according to any one of the preceding claims, cured to form an elastomer.

14. Water treatment process, characterised in that a suitable amount of an elastomer as defined in Claim 13 is immersed with a view to liberating a continuous and controlled amount of iodine into the water.